# EUROPEAN PATENT SPECIFICATION

(11) **EP 0 739 879 B1**
(45) Date of publication and mention of the grant of the patent: **22.12.1999**
(21) Application number: 96106361.7
(22) Date of filing: 23.04.1996
(51) Int. Cl.: C07C 261/02, C08L 63/00, C08L 79/08

(54) **Halogen-containing cyanate ester, process for producing the same and thermosetting resin composition containing the same**
Halogenenthaltende Cyanatester, Verfahren zu ihrer Herstellung und wärmehärtbare Harzzusammensetzungen, die sie enthalten
Esters de cyanate, procédé de leur préparation et compositions de résines thermodurcissables les contenant

(30) Priority: 24.04.1995 JP 9835795; 24.08.1995 JP 21604395
(43) Date of publication of application: 30.10.1996
(73) Proprietor: SUMITOMO CHEMICAL COMPANY LIMITED, Osaka-shi, Osaka 541-0041 (JP)
(72) Inventor: Watabu, Hisashi, Tsukuba-shi, Ibaraki (JP); Hayashi, Toshiaki, Tsukuba-shi, Ibaraki (JP); Shibata, Mitsuhiro, Tsukuba-gun, Ibaraki (JP)
(74) Representative: Henkel, Feiler, Hänzel

(56) References cited:
- DE-A- 2 529 487
- DE-A- 2 612 312
- DE-B- 1 195 764
- DE-B- 2 446 004
- US-A- 3 553 244

## Description

The present invention relates to a process for producing a halogen-containing cyanate ester and a flame-retardant thermostetting resin composition containing the same which is useful as encapsulated material or laminated plate for electronic parts, paint, composite, molding material and adhesive.

### BACKGROUND OF THE INVENTION

A thermosetting resin has widely been used as encapsulating material or laminated plate for electronic parts, paint, composite, molding material and adhesive in view of it's excellent characteristics and easy handling. Recently, as demands for responsibility for products become greater, a flame retardance of the thermosetting resin composition has become an essential element. On the other hand, with the arrival of high information age, low dielectric property of the resin has been required for the purpose of improving a signal propagation velocity, together with heat resistance in the use for electronic parts especially in case of resin for laminated plate.

As the thermosetting resin which satisfies the heat resistance and low dielectric simultaneously, a cyanate resin JP-A-50-129700 was proposed, but the cyanate resin itself was not flame-retardant. In order to give a cyanate resin flame-retardancy, it is known to use a flame-retardant epoxy resin as a thermosetting resin. However, it has been found by the results of the present inventors' study that the above cyanate resin causes deterioration of the heat resistance and exerts a deleterious influence on dielectric characteristics.

An attempt of producing the halogen-containing cyanate ester has been made. However, an acidity of a phenol group in which a halogen atom is substituted on a phenolic aromatic ring, becomes higher than that of a phenol group in which no halogen atom is substituted on a phenolic aromatic ring. As a result, the unreacted phenol group reacts with the formed cyanate group to give an iminocarbonate ester. When this iminocarbonate ester is present in the cyanate ester, the gelation time in thermosetting becomes short to deteriorate processability. Furthermore, the iminocarbonate ester contained in the formed cyanate resin absorbs moisture which causes hydrolysis and results in decarboxylation.

As the process for producing the halogen-containing cyanate ester by inhibiting the production of the iminocarbonate ester as a by-product, a process of cyanating a mixture of bisphenol A and a halogen-containing phenol is proposed, but it has not succeeded in isolating a halogen-containing cyanate ester in high purity (JP-B-61-54816).

### OBJECTS OF THE INVENTION

One object of the present invention is to provide a process for producing a halogen-containing cyanate ester of high purity serving as a flame retardant which is useful as encapsulating material or laminated plate for electronic parts, paint, composite, molding material and adhesive.

Another object of the present invention is to provide a flame-retardant thermosetting resin composition containing said halogen-containing cyanate ester or a prepolymer thereof which is superior in heat resistance and low dielectric.

These objects as well as other objects and advantages of the present invention will becomes apparent to those skilled in the art from the following description.

### SUMMARY OF THE INVENTION

The present invention provides a process for producing a halogen-containing cyanate ester by cyanating a phenol represented by the following general formula (1): 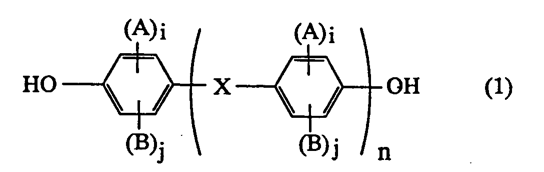 wherein A represents a hydrocarbon group having 1 to 6 carbon atoms; B represents a halogen atom; X represents a single bond, a hydrocarbon group having 1 to 20 carbon atoms, a carbonyl group, a sulfon group, a divalent sulfur atom or an oxygen atom; i represents an integer of 0 to 3; j represents an integer of 1 to 4; and n represents 0 or 1 with a cyanogen halide in the presence of a base comprising a nitrogen-containing heterocyclic aromatic compound, wherein the ester obtained has a value obtained by dividing a maximum value of an absorbance at a wave number of 1600 to 1740 cm⁻¹ by a maximum value of an absorbance at a wave number of 2100 to 2300 cm⁻¹ is not more than 0.2, the absorbance of a 1.0 % (weight by volume) chloroform solution of the halogen-containing cyanate esters being measured at a cell length of 1.0 mm using an infrared spectrophotometer.

The present invention also provides a thermosetting resin composition comprising such a halogen-containing cyanate ester or a prepolymer thereof a thermosetting resin and a curing agent. The term 'prepolymer(s)' as employed herein means that the compound has been homooligomerized or cooligomerized or interoligomerlized or homopolymerized or copolymerized or interpolymerized so as to cause an increase in molecular weight, but not to such an extent that the product has become cured, i.e. insoluble and infusible, but rather, the product is capable of being subsequently cured to an insoluble, infusible state.

### DETAILED DESCRIPTION OF THE INVENTION

The phenols used may be any one which satisfies the general formula (1). Examples of A include saturated or unsaturated hydrocarbon groups such as methyl group, ethyl group, isopropyl group, t-butyl group, cyclohexyl group, allyl group and phenyl group, and examples of B include halogen atoms such as chlorine and bromine. In case that two or more substituents A are contained in the formula (1), A may be the same or different from each other.

When n is 1, examples of the X include biphenyl type single bond, isopropylidene group, cyclohexylidene group, vinylene group, carbonyl group, ether group, sulfide group, sulfon group.

The phenols to be used may be any one which satisfies the general formula (1), and examples thereof include chlorophenol, bromophenol, 4-methyl-2-chlorophenol, 2-methyl-4-chlorophenol, 4-methyl-2-bromophenol, 2-methyl-4-bromophenol, dichlorophenol, dibromophenol, 4-methyl-2,5-dichlorophenol, 4-methyl-2,5-dibromophenol, tribromophenol, 3,3',5,5'-tetrachloro-4,4'-dihydroxybipheny 1, 3,3',5,5'-tetrabromo-4,4'-dihydroxybiphenyl, bis(3,5-dichloro-4-hydroxyphenyl)methane, bis(3,5-dibromo-4-hydroxyphenyl)methane, bis(3,5-dichloro-4-hydroxyphenyl)ethane, bis(3,5-dibromo-4-hydroxyphenyl)ethane, 2,2-bis(3,5-dichloro-4-hydroxyphenyl) propane, 2,2-bis(3,5-dibromo-4-hydroxyphenyl)propane, 1,1-bis(3,5-dichloro-4-hydroxyphenyl)cyclohexane, 1,1-bis(3,5-dibromo-4-hydroxyphenyl)cyclohexane, 1,1-bis(3,5-dichloro-4-hydroxyphenyl)butane, 1,1-bis(3,5-di bromo-4-hydroxyphenyl)butane, 1,1-bis(3,4-hydroxyphenyl)men thane, bis(3,5-dichloro-4-hydroxyphenyl)tricyclo[5,2,1,0^{2,6}] decane, 1,1-bis(3,4-hydroxyphenyl)methane, bis(3,5-dibromo-4-hydroxyphenyl)tricyclo[5,2,1,0^{2,6}]decane, bis(3,5-dichloro-4-hydroxyphenyl)sulfide, bis(3,5-dibromo-4-hydroxyphenyl)sulfide, bis(3,5-dichloro-4-hydroxyphenyl)ether, bis(3,5-dibromo-4-h ydroxyphenyl)ether, bis(3,5-dichloro-4-hydroxyphenyl)carbon yl, bis(3,5-dibromo-4-hydroxyphenyl)carbonyl, bis(3,5-dichloro-4-hydroxyphenyl)sulfon, bis(3,5-dibromo-4-hydroxyphenyl)sulfon.

The amount of the halogen-containing cyanate ester in the composition is not specifically limited as for as the thermosetting resin obtained satisfies the US flame retardance test (UL-94), and the desirable halogen amount of said cyanate ester in the thermosetting resin is 1-80% by weight to attain high heat-resistance and low dielectric. As the halogen in the cyanate ester, bromine is preferred.

In the thermosetting resin containing an epoxy resin and/or a cyanate ester, preferred bromine content is 10-30% by weight.

Moreover, a part of the halogen-containing cyanate ester can be replaced by a halogen-containing phenol or an epoxy compound thereof unless the heat resistance and dielectric are deteriorated.

The halogen-containing cyanate ester of the present invention can be obtained by using a nitrogen-containing heterocyclic aromatic compound a base when phenols are cyanated with a cyanogen halide in an organic solvent. In this case a high-purity halogen-containing cyanate ester can be obtained. Examples thereof include pyridine, lutidine, picoline and quinoline.

These bases may be used in combination in any proportion.

As the organic solvent, there can be used ketone solvents such as acetone, methyl ethyl ketone, methyl isobutyl ketone ; aromatic solvents such as benzene, toluene, xylene , ether solvents such as diethyl ether, methyl cellosolve, diglyme, tetrahydrofuran, dioxane ; hydrogen halides such as methylene chloride, chloroform, carbon tetrachloride, chlorobenzene ; alcohol solvents such as methanol, ethanol, isopropanol, methyl cellosolve, propylene glycol monomethyl ether ; aprotic solvents such as N,N'-dimethylformamide, N,N'-dimethylacetamide, N-methylpyrrolidone, 1,3-dimethyl-2-imidazolidone, dimethyl sulfoxide ; nitrile solvents such as acetonitrile, benzonitrile ; nitro solvents such as nitromethane, nitrobenzene ; ester solvents such as ethyl acetate, ethyl benzoate.

As the cyanogen halide, cyanogen chloride or cyanogen bromide is used. In case of using cyanogen chloride, the reaction temperature is -30 to 15°C, preferably -10 to 15°C. In case of using cyanogen bromide, the reaction temperature is -30 to 65°C. The amount of the nitrogen-containing heterocyclic aromatic compound may be 1.0- to 5-fold equivalent, preferably 1.0- to 1.5-fold equivalent, based on the equivalent of the hydroxyl group of the phenols. The amount of the cyanogen halide may be 1.0- to 5-fold equivalent, preferably 1.0- to 1.5-fold equivalent, based on the equivalent of the hydroxyl group of the phenols.

The post treatment is conducted by concentration, precipitation or crystallization after removing the hydrogen halide salt of the base formed as the by-product by washing with water or filtration. When it is washed with water, it is preferred to previously use a solvent causing phase separation from water as the reaction solvent. It is also possible to wash the reaction solution with an aqueous acid solution (e.g. hydrochloric acid, etc.) so as to wash the base efficiently. It is preferred that the concentration is conducted at the temperature lower than a melting point of the product under reduced pressure. When the temperature is too high, trimerization of the cyanate group initiates, and it is not preferred. The precipitation and crystallization are conducted by adding the product to a poor solvent, or adding the poor solvent to the product. Preferred examples of the poor solvent include alcohol solvents such as methanol, ethanol, isopropanol, amyl alcohol, t-butyl alcohol ; hydrocarbons such as benzene, toluene, xylene, hexane, petroleum ether . Alcohols may be used in combination with water in any proportion. In addition, alcohols and hydrocarbons nay be used in combination in any proportion.

The purity of the resulting halogen-containing cyanate ester can be quantatively determined by an infrared spectrophotometer. That is, the adsorption band of the halogen-containing cyanate ester is observed at 2100 to 230 cm⁻¹ while the absorption band of the iminocarbonate ester as the impurity is observed at 1660 to 1740 cm⁻¹ so that the purity can be determined by the intensity ratio. When the infrared absorption spectrum of the halogen-containing cyanate ester obtained according to the present invention is measured, a value obtained by dividing a maximum value of an absorbance at a wave number of 1600 to 1740 cm⁻¹ by a maximum value of an absorbance at a wave number of 2100 to 2300 cm⁻¹ is not more than 0.2.

The above-described absorbance can be determined as follows. That is, a 1.0 % (weight by volume) chloroform solution of the halogen-containing cyanate ester is charged in a KBr cell for liquid (cell lengh: 1.0 mm), and then an infrared spectrum (total number of determinations: 4) is measured by a Fourier transform infrared spectrophotometer (e.g. Perkin Elmer® 1600 type, manufactured by Perkin Elmer Japan Co.) to determine the maximum value of the absorbance at the wave number of 1600 to 1740 cm⁻¹ and that at the wave number of 2100 to 2300 cm⁻¹ in comparison with a blank (chloroform).

When the halogen-containing cyanate ester is synthesized by using triethylamine as the base according to the process for synthesizing a cyanate ester which has hitherto been known (U.S. Patent No. 3,553,244) and the infrared absorption spectrum of the resulting halogen-containing cyanate ester is measured by the above process, the intensity ratio becomes 1.22. The halogen-containing cyanate ester containing a large amount of the iminocarbonate ester is gelled at 200°C within 30 seconds, and is not suitable for practical use.

According to the results of the present inventors' study, the gelation time at 200°C of the halogen-containing cyanate ester having an intensity ratio of not more than 0.2 (measured under the same condition) becomes not less than 60 seconds, and it can be put to practical use.

The thermosetting resin used in the present invention is not specifically limited, and examples thereof include epoxy resin, thermosetting polyimide resin, phenol resin, resol resin, urethane resin, vinyl group-containing compound, allyl group-containing compound, propargyl group-containing compound, cyanate ester . They may be optionally used in combination thereof.

Among them, thermosetting polyimide resin and halogen-free cyanate esters are particularly preferred because they provide halogen-containing cyanate esters and a resin composition having high heat resistance and low dielectric.

The halogen-free cyanate esters used in the present invention are cyanate esters having a plurality of cyanate groups in the molecule, and those obtained by any methods can be used.

Examples of the halogen-free cyanate esters which are preferred for the objects of the present invention include any cyanate esters such as 4,4'-dicyanatebiphenyl, 3,3',5,5'-tetramethyl-4,4'-dicyanatebiphenyl, bis(cyanatephenyl)methane, bis(4-cyanate-3,5-dimethylphenyl) methane, bis(4-cyanate-3-t-butyl-2-methylphenyl)methane, bis(4-cyanatephenyl)ethane, 2,2-bis(4-cyanatephenyl)propane , 2,2-bis(3,5-dimethyl-4-cyanatephenyl)propane, 2,2-bis(3-methyl-4-cyanatephenyl)propane, 2,2-bis(4-cyanate-3-t-butylphenyl)propane, 2,2-bis(4-cyanate-3-t-butyl-6-methylphenyl)propane, 2,2-bis(4-cyanate-3-allylphenyl)propane, 1,1-bis(4-cyanate-3-t-butyl-6-methylphenyl)butane, 1,1-bis(4-cyanatephenyl)cyclohexane, 1,1-bis(4-cyanate-3-methylphenyl)cyclohexane, 1,1-bis(4-cyanate-3-cyclohexyl-6-methyl)butane, bis(4-cyanatephenyl)menthane, bis(4-cyanate-3,5-dimethyl)menthane, bis(4-cyanate-3-t-butyl-6-methylphenyl)menthane, bis(4-cyanatephenyl)tricyclo[5,2,1,0^{2,6}]decane, bis(4-cyanate-3,5-dimethylphenyl)tricyclo[5,2,1,0^{2,6}]decane, bis(4-cyanate-3-t-butyl-6-methylphenyl)tricyclo[5,2,1,0^{2,6}]decane, bis(4-cyanatephenyl)sulfide, bis(4-cyanate-3,5-dimethyllphenyl)sulfide, bis(4-cyanate-3-t-butyl-6-methylphenyl)sulfide, bis(4-cyanatephenyl)sulfon, bis(4-cyanatephenyl)carbonyl, bis(4-cyanatephenyl)ether.

They may be used in combination thereof.

Among them, 2,2-bis(4-cyanatephenyl)propane is particularly preferred because it is superior in heat resistance and is easily available. In addition, 1,1-bis(4-cyanate-3-t-butyl-6-methylphenyl)butane is preferred because it is superior in low electric and shows high heat resistance and low moisture absorption properties.

The epoxy resin used in the present invention is not specifically limited, and examples thereof include glycidyl etherified products of alkyl phenolic novolaks such as diglycidyl ether of bisphenol A and bisphenol E, glycidyl etherified product of phenolic novolak and cresol novolak, tetrabromobisphenol A type epoxy resin, glycidyl etherified product of brominated phenolic novolak, 4-octyl phenolic novolak, 2-t-butyl-5-methyl phenolic novolak and polyfunctional epoxy resins. They may be optionally used in combination thereof.

The thermosetting polyimide resin used in the present invention may be polyfunctional maleimide having two or more N-maleimide groups in the molecule, maleimide polymer, or prepolymer of maleimide and amine. Examples thereof include 4,4'-diaminodiphenylmethane bismaleimide and an addition polymerized product of 4,4'-diaminodiphenylmethane bismaleimide and 4,4'-diaminodiphenylmethane. They may be optionally used in combination thereof.

The thermosetting resins used in the present invention can be used in combination in any proportion unless the flame retardance is deteriorated.

It is confirmed that all Examples described hereinafter show V-0 class flame retardance by the test conducted according to UL-94 (U.S.A. flame retardance test). However, it is also possible to produce a flame-retardant resin composition wherein the bromine content is further reduced according to the environment to be used.

As the curing agent of the resin composition of the present invention, known cyanate esters and other thermosetting resins can be used. Examples of the cyanate ester as the crosslinking agent include protonic acids such as hydrochloric acid, phosphoric acid ; Lewis acids such as aluminum chloride, boron trifluoride complex, zinc chloride ; aromatic hydroxy compounds such as phenol, pyrocatechol, nonylphenol, dihydroxynaphthalene ; organic metal salts such as zinc naphthenate, cobalt naphthenate, tin octylate, cobalt octylate ; metal salts such as zinc acetylactonate, copper acetylacetonate, cobalt acetylacetonate ; tertiary amines such as triethylamine, tributylamine, pyridine, quinoline ; imidazoles; quaternary ammonium salts such as tetraethylammonium chloride, tetramethylammonium bromide, benzylammonium chloride ; metal hydroxides such as sodium hydroxide ; metal alcoholates such as sodium methoxide ; triphenylphosphine; diazabicyclo-(2,2,2)-octane . Among them, naphthenic acid metal salts and octanoic acid metal salts are particularly preferred because the thermosetting rate is fast and they are easily available.

As for the thermosetting resin, known curing agents can be used unless the objective characteristics are not damaged. In case of the epoxy resin, examples of the curing agent include imidazoles, amines, acid anhydrides, phosphorous compounds, phenolic novolaks .

In the present invention, known additives such as flame retardants, releasants, surface treating agents, fillers may be added to the composition according to the application.

Examples of the retardant include antimony trixide, red phosphorous flame retardant, bromine-containing flame retardant . Examples of the releasant include waxes, zinc stearate . Examples of the surface treating agent include silane coupling agent . Examples of the filler include silica, alumina, talc, clay, glass fiber .

A laminated plate can be obtained by dissolving the thermosetting resin composition of the present invention in an organic solvent, impregnating a substrate of a woven fabric, mat or paper of organic or inorganic fibers (e.g. glass fibers, polyester fibers, alumina fibers ) or a combination thereof with the impregnating solution, drying the impregnated substrate with heating to form a prepreg, followed by hot-press molding. The organic solvent which can be used is not specifically limited, and examples thereof include methyl ethyl ketone, methyl isobutyl ketone, ethylene glycol monomethyl ether, propylene glycol dimethyl ether, toluene, xylene, dioxane, tetrahydrofuran, dimethylformamide, N-methylpyrrolidone . They may be optionally used in combination thereof.

The following Examples and Comparative Examples further illustrate the present invention in detail but are not to be construed to limit the scope thereof. In the Examples and Comparative Examples, the epoxy equivalent is defined by the molecular weight of the epoxy resin per one epoxy group, and OH equivalent is defined by the molecular weight of the polyphenol compound per one OH group.

### Synthesis Example

This Synthesis Examples relates to a process for producing glycidyl ether of novolak of 2-t-butyl-5-methylphenol as the raw material of the epoxy resin used for the epoxy resin composition of the present invention.

2-t-Butyl-5-methylphenol (2231.0 g, 13.58 OH mol), p-toluenesulfonic acid (12.9 g, 0.068 mol) and deionized water (223.2 g) were charged in a 5 liter four-necked round flask equipped with a thermometer, a stirrer, a cooling tube and a dropping tube, and the mixture was heated to 100°C. After 37 % formalin (218.4 g, 2.715 mol) was added dropwise over 2 hours, the reaction was conducted while maintaining at 100°C for 2 hours. The reaction solution was cooled to 80°C, and then neutralized with an aqueous 10 % NaOH solution (27.7 g, 0.069 mol). The organic layer after partitioning was washed twice with deionized water (700 g). The organic layer after washing was concentrated under reduced pressure (180°C/10 mmHg/one hour) to obtain a resinous product. The OH equivalent of the resulting resinous product was 176.0 g/eq.

The reaction product (246.4 g, 1.4 OH mol) obtained as described above, epichlorohydrin (906.5 g, 9.8 mol), dimethyl sulfoxide (453.3 g) and deionized water (14.0 g) were charged in a 2 liter four-necked round flask equipped with a thermometer, a stirrer and a cooling tube with a column, and an aqueous 48.6 % sodium hydroxide solution (108.31 g, 1.316 mol) was added dropwise under the condition of 49°C and 42 torr over 5 hours, whereas azetropic epichlorohydrin and water were liquefied with cooling while maintaining the temperature at 49°C and the reaction was conducted while returning the organic layer to the reaction system.

After the completion of the reaction, the nonreacted epichlorohydrin was removed by concentration under reduced pressure and, after dissolving an epoxidated product containing a by-product salt and dimethyl sulfoxide in methyl isobutyl ketone, the by-product salt and dimethyl sulfoxide were removed by washing with hot water. The solvent was removed under reduced pressure to obtain 304.9 g of an epoxy resin.

The epoxy equivalent of the resulting epoxy resin was 256 g/eq. The infrared absorption spectrum was measured. As a result, the absorption at 3200 to 3600 cm⁻¹ of a phenolic OH disappeared and it was confirmed that the resulting epoxy resin has the absorptions at 1240, 910 cm⁻¹ of epoxide.

### Example 1

2,2-Bis(3,5-dibromo-4-hydroxyphenyl)propane ( manufactured by Bromino Compounds Co.) (108.8 g, 0.2 mol), methylene chloride (643 g) and pyridine (guaranteed, Wako Junyaku Co., Ltd.) (44.3 g, 0.56 ml) were charged in a round flask equipped with a stirrer, and the mixture was stirred, dissolved and then cooled to 0°C. In addition, methylene chloride (50 g) was charged in a reaction vessel equipped with a thermometer, a stirrer, a dropping funnel and a reflux condenser under a nitrogen atmosphere and, after cooling to 0°C, cyanogen chloride (49.2 g, 0.8 mol) was added. The solution in the round flask was added dropwise to this reaction vessel at -2 to 6°C over 2 hours and 30 minutes, followed by maintaining at the same temperature for 30 minutes. Then, the reaction solution heated to room temperature was washed with an aqueous 3 % hydrochloric acid (300 g), washed twice with water, and then partitioned. The resulting organic layer was concentrated to about 200 g under reduced pressure and, after methanol (200 ml) was added dropwise, the mixture was stirred with cooling to 5°C for 3 hours. The resulting slurry was filtered, washed with methanol (350 ml), and then air-dried to obtain 103.3 g of a pale yellow crystal having a melting point of 199°C (yield: 88 %). The area (%) of the raw bisphenol, monocyanate and dicyanate was respectively analyzed by LC (liquid chromatography). As a result, the area (%) of the dicyanate was 99 %. In addition, a 1.0 % (weight by volume) chloroform solution of this crystal was charged in a KBr cell for liquid (cell length: 1.0 mm), and then an infrared spectrum (4 accumulative calculations) was measured by a Fourier transform infrared spectrophotometer (Perkin Elmer® 1600 type, manufactured by Perkin Elmer Japan Co.). As a result, the absorption at 1740 to 1660 cm⁻¹ of the iminocarbonate ester group was not observed.

### Example 2

2,2-Bis(3,5-dibromo-4-hydroxyphenyl)propane ( manufactured by Bromino Compounds Co.) (108.8 g, 0.2 mol) and methyl isobutyl ketone (643 g) were charged in a round flask equipped with a stirrer, a dropping funnel and reflux condenser under a nitrogen atmosphere, followed by cooling to 0°C. Pyridine (guaranteed, Wako Junyaku Co., Ltd.) (44.3 g, 0.56 mol) and cyanogen chloride (49.2 g, 0.8 mol) were added dropwise to this reaction solution at -2 to 6°C over 2 hours and 30 minutes, followed by maintaining at the same temperature for 30 minutes. Then, the resulting slurry was added to isopropanol (500 ml) cooled to -5°C and, after stirring with cooling at the ice-cold temperature for 30 minutes, the mixture was allowed to stand. The resulting precipitate was filtered together with the solution, and then washed with isopropanol (500 ml). After air-drying, 84.3 g of a pale yellow crystal having a melting point of 197°C was obtained (yield: 71 %). The area (%) of the dicyanate due to LC was 92 %. In addition, the absorption at 1660 to 1740 cm⁻¹ was not observed in the infrared spectrum of this crystal.

### Comparative Example 1

2,2-Bis(3,5-dibromo-4-hydroxyphenyl)propane ( manufactured by Bromino Compounds Co.) (108.8 g, 0.2 mol) and methyl isobutyl ketone (643 g) were charged in a round flask equipped with a stirrer, a dropping funnel and reflux condenser under a nitrogen atmosphere, followed by cooling to 0°C. Then, triethylamine (guaranteed, Wako Junyaku Co., Ltd.) (44.5 g, 0.44 mol) and cyanogen chloride (27.1 g, 0.44 mol) were added dropwise to this reaction solution at -2 to 6°C over 2 hours and 30 minutes, followed by maintaining at the same temperature for 30 minutes. After the reaction solution heated to room temperature was washed with an aqueous 3 % hydrochloric acid (300 g) and then washed twice with water (300 g), the solvent was concentrated under reduced pressure and cooled to 5°C. The resulting product was added to methanol cooled to 5°C and, after stirring with cooling at the ice-cold temperature for 30 minutes, the mixture was allowed to stand. The resulting precipitate was filtered together with the solution, and then washed with methanol (500 ml). After air-drying, 90.7 g of a white crystal having a melting point of 187°C was obtained (yield: 77 %). The raw bisphenol and monocyanate were not detected by the analysis due to LC. After analyzing by GPC (gel permeation chromatography), the area (%) of the monomer was only 40 % and others were oligomers whose molecular weight is larger than that of the objective cyanates. The value of this crystal, obtained by dividing a maximum value of an absorbance at a wave number of 1600 to 1740 cm⁻¹ by a maximum value of an absorbance at a wave number of 2100 to 2300 cm⁻¹, was 1.22. The gelation time of this white crystal at 200°C was 30 seconds.

A predetermined amount of the pale yellow crystal obtained in Example 1 and white crystal obtained in Comparative Example 1 were mixed, dissolved in chloroform, and then air-dried to obtain a sample. The intensity ratio obtained from the infrared absorption spectrum and gelation time at 200°C of the resulting sample are shown in Table 1.

As shown in Table 1, the gelation time at 200°C of the halogen-containing cyanate ester having the intensity ratio (due to infrared absorption spectrum) of not more than 0.2 is not less than 60°C and, therefore, it can be put to practical use.

**Table 1**

| | Example 1 | Example 3 | Example 4 | Example 5 | Comparative Example 2 | Comparative Example 1 |
|---|---|---|---|---|---|---|
| dicyanate of Example 1 (parts by weight) | 100 | 95 | 90 | 75 | 50 | 0 |
| dicyanate of Comparative Example 1 (parts by weight) | 0 | 5 | 10 | 25 | 50 | 100 |
| Intensity ratio of infrared absorption spectrum ^{a)} | c) | 0.03 | 0.08 | 0.16 | 0.44 | 1.22 |
| Gelation time (second) ^{b)} | d) | 640 | 452 | 193 | 51 | 30 |

| | | | | | | |
|---|---|---|---|---|---|---|
| (a) (Maximum value of absorbance at wave number of 1600 to 1740 cm⁻¹)/(maximum value of absorbance at wave number of 2100 to 2300 cm⁻¹) | | | | | | |
| (b) Measured at 200°C using a gelation testing equipment (GT-D type, manufactured by Nisshin Kagaku Co., Ltd.). | | | | | | |
| (c) The absorption at 1660 to 1740 cm⁻² was not observed. | | | | | | |
| (d) Not gelled even after 1000 seconds. | | | | | | |

### Examples 6 to 14

A thermosetting resin (predetermined parts by weight described in Table 2) was added to tetrabromobisphenol A dicyanate obtained according to Example 1, followed by substituting with nitrogen. Then, the mixture was heated to 140°C (bulk temperature), stirred for 2 hours and cooled to give a 50 wt % solution. When the mixture was not dissolved by heating to 140°C after mixing, dimethylformamide was added to give a 50 wt % solution which was stirred at 140°C for 2 hours, and then cooled. The curing agent described in Table 1 was added to the resulting organic solution and, after forming a prepolymer at 170°C, the prepolymer was pressed under the condition of 200°C/50 kg/cm² for one hour and postcured at 200°C for 2 hours to obtain a cured product having a thickness of about 2 mm.

The glass transition temperature and dielectric constant of the cured products obtained in Examples 6 to 14 are respectively shown Table 2.

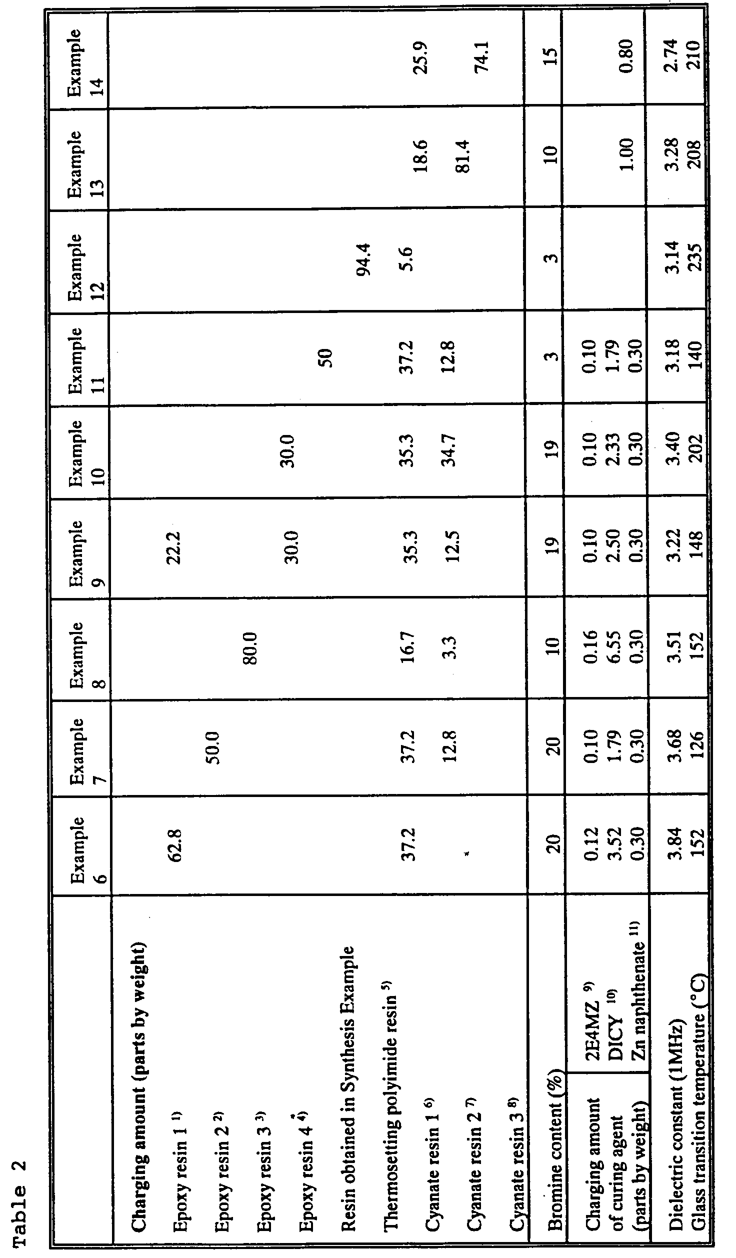

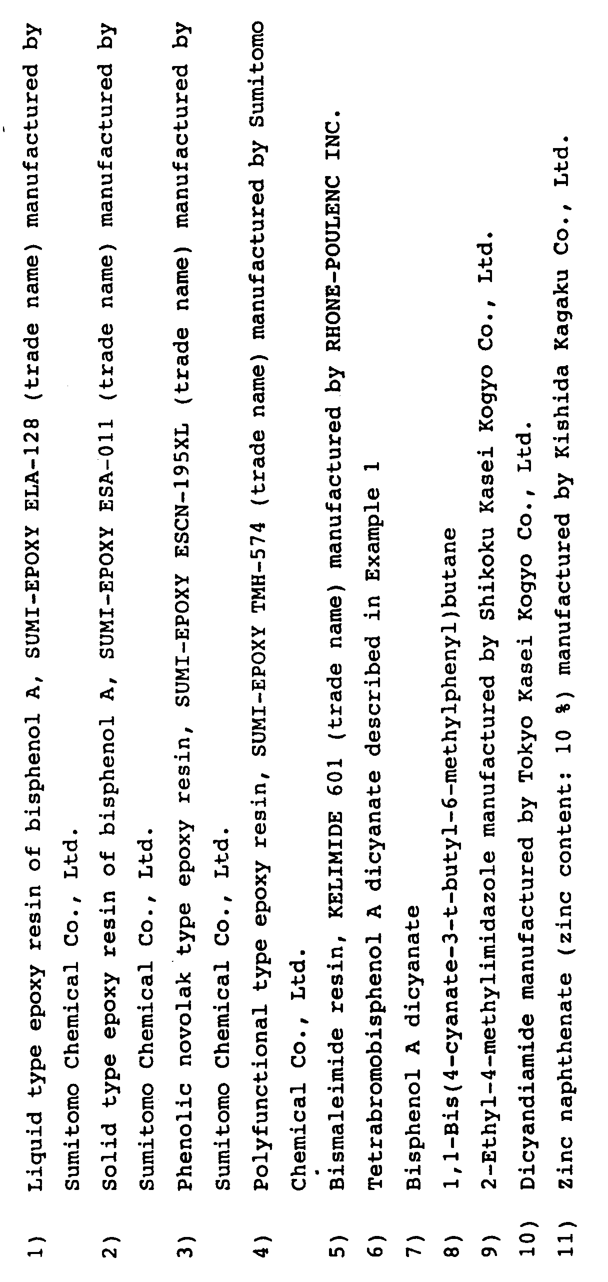

### Comparative Examples 3 to 10

Comparative Examples 3 to 10 relate to a cured product of a resin composition using a cured product of a thermosetting resin and a bromine-containing flame retardant other than halogen-containing cyanate ester.

According to the same manner as that described in the above-described Examples, a cured product having a thickness of about 2 mm was produced by formulating the resin and curing agent described in Table 3. These cured products (excluding those containing no flame retardant of Comparative Examples 4 and 5) were subjected to a flame retardance test according to U.S.A flame resistance test specification UL-94. As a result, all cured products showed V-0 class.

The glass transition temperature and dielectric constant of the resulting cured products are respectively shown in Table 3.

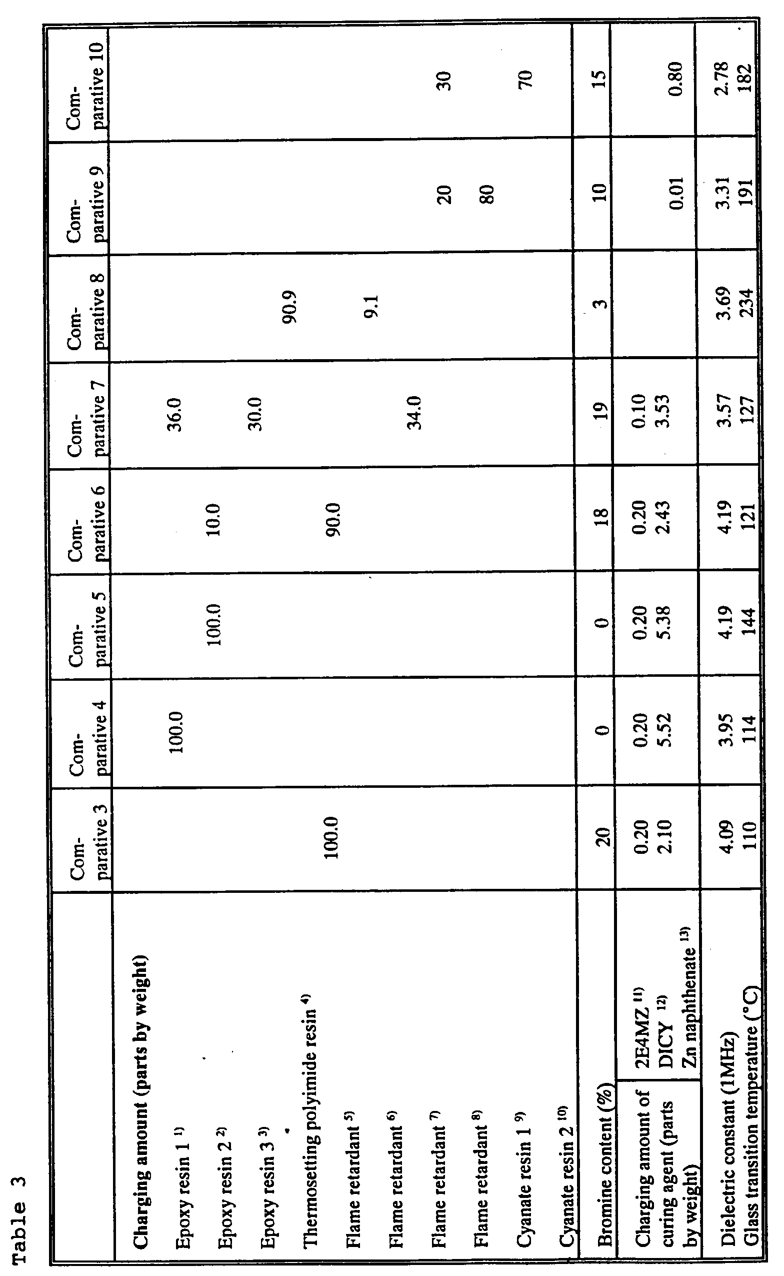

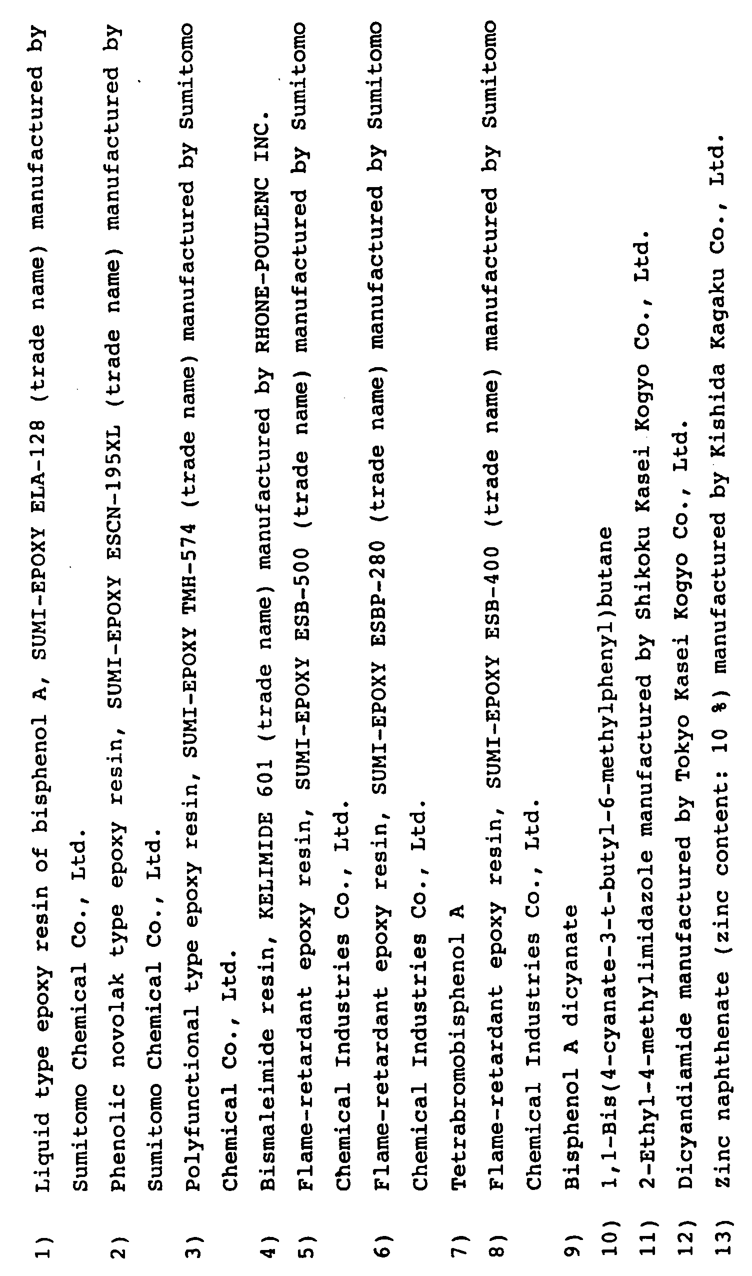

Further, physical properties were measured by the following method in Tables 2 and 3.

The glass transition temperature was measured by a thermomechanical analytical device TMA 120 manufactured by Seiko Denshi Kogyo Co., Ltd.

The dielectric constant was determined as follows. That is, an electrode was formed on both surfaces of a cured product having a thickness of about 2 mm using a metal paint, and then the dielectric constant was calculated from a value of an electrostatic capacity obtained by using a Multi-Frequency LCR Meter 4275A (manufactured by YHP Co., Ltd.).

It is apparent from the results of the Examples and Comparative Examples that the cured products of the thermosetting resin composition containing halogen-containing cyanate esters of the present invention shows low electric and high glass transition temperature.

As is shown in the Comparative Examples, the halogen-containing cyanate ester is immediately gelled and, therefore, it is difficult to put to practical use according to a conventional invention. However, regarding high-purity halogen-containing cyanate ester obtained according to the present invention, the gelation time is easily adjusted by adding a curing catalyst because the gelation times is slow. Accordingly, it became possible to provide a halogen-containing cyanate ester serving as a flame retardant which is useful as encapsulating material or laminated plate for electronic parts paint, composite, molding material and adhesive. Furthermore, there can be obtained a flame-retardant cured product having excellent heat resistance and low dielectric by using the thermosetting resin composition.

## Claims

1. A process for producing halogen-containing cyanate esters, wherein a phenol represented by the following general formula (1): 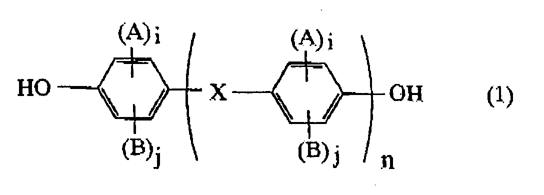 wherein A represents a hydrocarbon group having 1 to 6 carbon atoms; B represents a halogen atom; X represents a single bond, a hydrocarbon group having 1 to 20 carbon atoms, a carbonyl group, a sulfon group, a divalent sulfur atom or an oxygen atom; i represents an integer of 0 to 3; j represents an integer of 1 to 4; and n represents 0 or 1
is cyanated with cyanogen halide in the presence of a base, comprising a nitrogen-containing heterocyclic aromatic compound,
wherein the ester obtained has a value obtained by dividing a maximum value of an absorbance at a wave number of 1600 to 1740 cm⁻¹ by a maximum value of an absorbance at a wave number of 2100 to 2300 cm⁻¹ is not more than 0.2, the absorbance of a 1.0% (weight by volume) chloroform solution of the halogen-containing cyanate esters being measured at a cell length of 1.0 mm using an infrared spectrophotometer.

2. The process for producting the halogen-containing cyanate esters according to Claim 1, wherein the phenol represented by the general formula (1)is a bisphenol represented by the following formula:

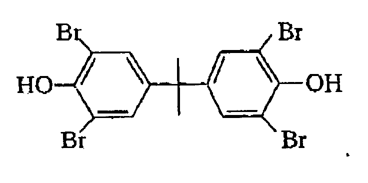

3. The process for producing halogen-containing cyanate esters according to claim 1, wherein pyridine, lutidine, picoline or quinoline is used as the nitrogen-containing heterocyclic aromatic compound.

4. A thermosetting resin composition comprising the halogen-containing cyanate esters of claim 1 or a prepolymer thereof, a thermosetting resin and a curing agent.

5. The thermosetting resin composition according to claim 4, wherein the thermosetting resin is a halogen-free cyanate ester or a prepolymer thereof.

6. The thermosetting resin composition according to claim 4, wherein the thermosetting resin is an epoxy resin.

7. The thermosetting resin composition according to claim 4, wherein the thermosetting resin is a thermosetting polyimide resin.

## Patentansprüche

1. Verfahren zur Herstellung halogenhaltiger Cyanatester, wobei ein Phenol der folgenden allgemeinen Formel (1): 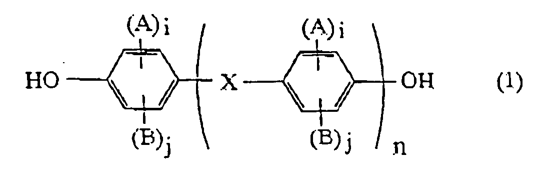 worin bedeuten:
A eine Kohlenwasserstoffgruppe mit 1 bis 6 Kohlenstoffatom(en);
B ein Halogenatom;
X eine Einfachbindung, eine Kohlenwasserstoffgruppe mit 1 bis 20 Kohlenstoffatom(en), eine Carbonylgruppe, eine Sulfongruppe, ein zweiwertiges Schwefelatom oder ein Sauerstoffatom;
i eine ganze Zahl von 0 bis 3;
j eine ganze Zahl von 1 bis 4, und
n 0 oder 1,
mit einem Halogencyan in Gegenwart einer Base, umfassend eine stickstoffhaltige heterocyclische aromatische Verbindung, cyaniert wird,
wobei der erhaltene Ester einen durch Dividieren des maximalen Extinktionswerts bei einer Wellenzahl von 1600 bis 1740 cm⁻¹ durch den maximalen Extinktionswert bei einer Wellenzahl von 2100 bis 2300 cm⁻¹ erhaltenen Wert von nicht mehr als 0,2 aufweist, wobei die Extinktion einer 1,0%igen (Gewicht/Volumen) Chloroformlösung der halogenhaltigen Cyanatester mittels eines Infrarotspektralphotometers bei einer Küvettenlänge von 1,0 mm gemessen wird.

2. Verfahren zur Herstellung der halogenhaltigen Cyanatester nach Anspruch 1, wobei das phenol der allgemeinen Formel (1) aus einem Bisphenol der folgenden Formel: 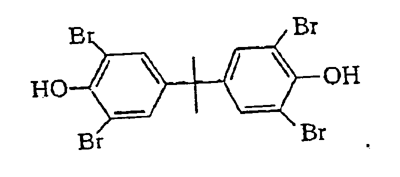 besteht.

3. Verfahren zur Herstellung halogenhaltiger Cyanatester nach Anspruch 1, wobei als Stickstoffhaltige heterocyclische aromatische Verbindung Pyridin, Lutidin, Picolin oder Chinolin verwendet wird.

4. Wärmehärtbare Harzmasse, umfassend die halogenhaltigen Cyanatester nach Anspruch 1 oder ein präpolymer hiervon, ein wärmehärtbares Harz und ein Härtungsmittel.

5. Wärmehärtbare Harzmasse nach Anspruch 4, wobei das wärmehärtbare Harz aus einem halogenfreien Cyanatester oder einem präpolymer hiervon besteht.

6. Wärmehärtbare Harzmasse nach Anspruch 4, wobei das wärmehärtbare Harz aus einem Epoxyharz besteht.

7. Wärmehärtbare Harzmasse nach Anspruch 4, wobei das wärmehärtbare Harz aus einem wärmehärtbaren polyimidharz besteht.

## Revendications

1. Procédé de production de cyanates halogénés, dans lequel on procède à la cyanatation d'un phénol représenté par la formule générale (1) suivante : 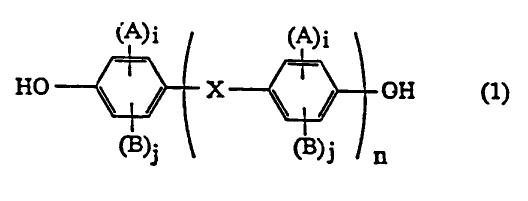 dans laquelle A représente un groupe hydrocarboné de 1 à 6 atomes de carbone; B représente un atome d'halogène; X représente une liaison simple, un groupe hydrocarboné de 1 à 20 atomes de carbone, un groupe carbonyle, un groupe sulfone, un atome de soufre divalent ou un atome d'oxygène ; i représente un entier de 0 à 3; j représente un entier de 1 à 4; et n représente 0 ou 1,
avec un halogénure de cyanogène en présence d'une base comprenant un composé aromatique hétérocyclique azoté,
et dans lequel le cyanate obtenu a une valeur, obtenue par division de la valeur maximale de l'absorbance à un nombre d'onde de 1600 à 1740 cm⁻¹ par la valeur maximale de l'absorbance à un nombre d'onde de 2100 à 2300 cm⁻¹, qui n'est pas supérieure à 0,2, l'absorbance d'une solution à 1,0 % (masse par volume) du cyanate halogéné dans le chloroforme étant mesurée à une longueur de cuve de 1,0 mm à l'aide d'un spectrophotomètre infrarouge.

2. Procédé de production de cyanates halogénés selon la revendication 1, dans lequel le phénol représenté par la formule générale (1) est un bisphénol représenté par la formule suivante :

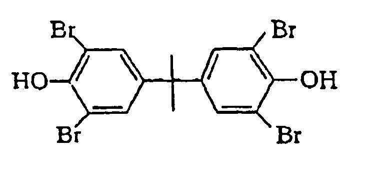

3. Procédé de production de cyanates halogénés selon la revendication 1, dans lequel on utilise de la pyridine, de la lutidine, de la picoline ou de la quinoléine comme composé aromatique hétérocyclique azoté.

4. Composition de résine thermodurcissable comprenant un cyanate halogéné selon la revendication 1 ou un de ses prépolymères, une résine thermodurcissable et un agent de durcissement.

5. Composition de résine thermodurcissable selon la revendication 4, dans laquelle la résine thermodurcissable est un cyanate non halogéné ou un de ses prépolymères.

6. Composition de résine thermodurcissable selon la revendication 4, dans laquelle la résine thermodurcissable est une résine époxy.

7. Composition de résine thermodurcissable selon la revendication 4, dans laquelle la résine thermodurcissable est une résine polyimide thermodurcissable.
